# EUROPEAN PATENT SPECIFICATION

(11) **EP 0 858 289 B1**
(45) Date of publication and mention of the grant of the patent: **28.06.2000**
(21) Application number: 95924468.2
(22) Date of filing: 12.07.1995
(51) Int. Cl.: A61B 5/15

(54) **BLOOD SAMPLING DEVICE**
BLUTENTNAHME-VORRICHTUNG
DISPOSITIF DE PRISE D'ECHANTILLON DE SANG

(30) Priority: 13.07.1994 GB 9414143
(43) Date of publication of application: 19.08.1998
(73) Proprietor: OWEN MUMFORD LTD., Woodstock Oxford OX7 1TU (GB)
(72) Inventor: MARSHALL, Jeremy, Oxford OX2 6DD (GB); CROSSMAN, David, Danvers, Oxford OX9 5HL (GB)
(74) Representative: Lainé, Simon James
(86) International application number: GB9501652
(87) International publication number: WO9602189

(56) References cited:
- FR-A- 2 595 237
- US-A- 4 889 117

## Description

This invention relates to blood sampling devices, and in particular to a lancet for pricking the skin to draw a small drop of blood for analysis. Such prickers are widely used by diabetics, for example, who need to know their sugar level. However, there are many other applications.

There is an obvious need to make the sharp tip of the lancet safe after use. There have been various proposals for having the lancet as part of a cheap, disposable device which, after a single use, can be thrown away with the lancet automatically retracted inside the body and thus made safe. These are very handy, but in the long run it is more expensive to use them than to have a permanent firing mechanism and to load it when required with a lancet. But then the lancet must be made safe after removal.

US-A-4889117 discloses a skin pricker with a needle encased in a tube, and the tip of the needle is projected forwards by a rear end cap, another cap at the forward end having been removed. After use, the needle is withdrawn back into the tube and the forward end re-capped. That is not a lancet of the kind which is loaded into a firing mechanism (it requires the user to push the rear end cap to prick the skin) and its state is the same after as before use. It can be used again and again, and is inherently unsafe.

FR-A-2595237 describes a lancet, but the tip that penetrates the skin is integrally moulded with the main body.

The usual form of lancet has a steel needle encased in an elongate plastics body. A cap is moulded integrally with this body and initially the tip of the needle is embedded in it. But the connection between the cap and the body is weak and the cap can be pulled free to expose the tip. After use, the cap may be used to cover the needle tip again, and it may be re-oriented to fit over the forward end of the body.

The needle tends to be smooth and the plastics material of the body does not have much grip on it. It can happen that, when breaking the cap free, the cap is squeezed or angled with respect to the body sufficiently to pull the needle forwardly by its tip with respect to the body. There is then a lancet with a needle tip extending too far.

There is also a trend towards making the needle thinner, to achieve an even sharper point, and therefore a less noticeable prick. But the needle also serves to give some strength to the lancet as a whole, providing a reinforcing rod along its axis. If it is too thin, the lancet is able to bend rather easily, and this is unsatisfactory.

It is the aim of aspects of this invention to remedy these faults.

According to the present invention there is provided a lancet for a skin pricker comprising a needle with a tip and an elongate body of moulded plastics material encasing most of the needle, which is movable rearwardly with respect to the body to retract the needle tip within the body after use, characterised in that the needle tip is initially encased by a breakaway cap integrally moulded with the body at its forward end and which is exposed when the cap is removed, in that the mutual engagement of the needle and body is such that the needle cannot move forwards from its initial position relative to the body and in that the needle and body remain effectively integral and move together during the act of skin pricking.

Thus, instead of trying to place the cap back over the end of the lancet after use, the needle is pushed backwards in relation to the body until the tip is flush with or recessed into the forward end.

In one preferred form the mutual engagement is a frictional grip. Alternatively, it can be a membrane at the rear end of the body against which the rear end of the needle bears during skin pricking, but which can be broken when the needle is urged rearwardly by a substantial force. In another form the mutual engagement is a throat at the rear end of the body in which the rear end of the needle is trapped during skin pricking, but through which that rear end of the needle can be urged by a substantial force.

Conveniently the cap is adapted to serve as an implement for pushing the tip of the needle, after use, back into the body. Preferably, the forward end of the body is recessed and the cap has a projection which can enter the recess when pushing the tip of the needle. Then, even if the tip is not pushed right home into the body, it will be out of harms way.

The rear end of the body is advantageously adapted to shield the rear end of the needle when the needle is retracted. This should prevent an accidental knock pushing the needle forwards again and re-projecting the tip.

The impediment to forward movement of the needle from its initial position relative to the body may simply be a forwardly facing shoulder where the needle reduces in cross-section to a leading length portion projecting from the body and terminating in the tip.

The needle may be generally cylindrical with a flat formed over the leading length portion to make the reduced cross-section.

The flat may be substantially diametral, and the shoulder at its rear end will provide an abutment preventing the needle from being pulled forwards when the cap is removed. But of course it will be no impediment to the needle being pushed rearwardly.

The flat also helps to achieve a really sharp point, with a cross-sectional size of a thin needle, but it leaves most of the length of the needle with its original cylindrical form, so that its reinforcing properties are not significantly impaired.

For a better understanding of the invention, one embodiment will now be described, by way of example, with reference to the accompanying drawings, in which:
Figure 1 is a side view of a lancet in a pre-use condition,
Figure 2 is another side view of the lancet viewed in the direction A of Figure 1, showing its encased needle.
Figure 3 is a rear end view of the lancet,
Figure 4 is a perspective view of the needle that forms part of the lancet, and
Figure 5 shows stages of use of the lancet.

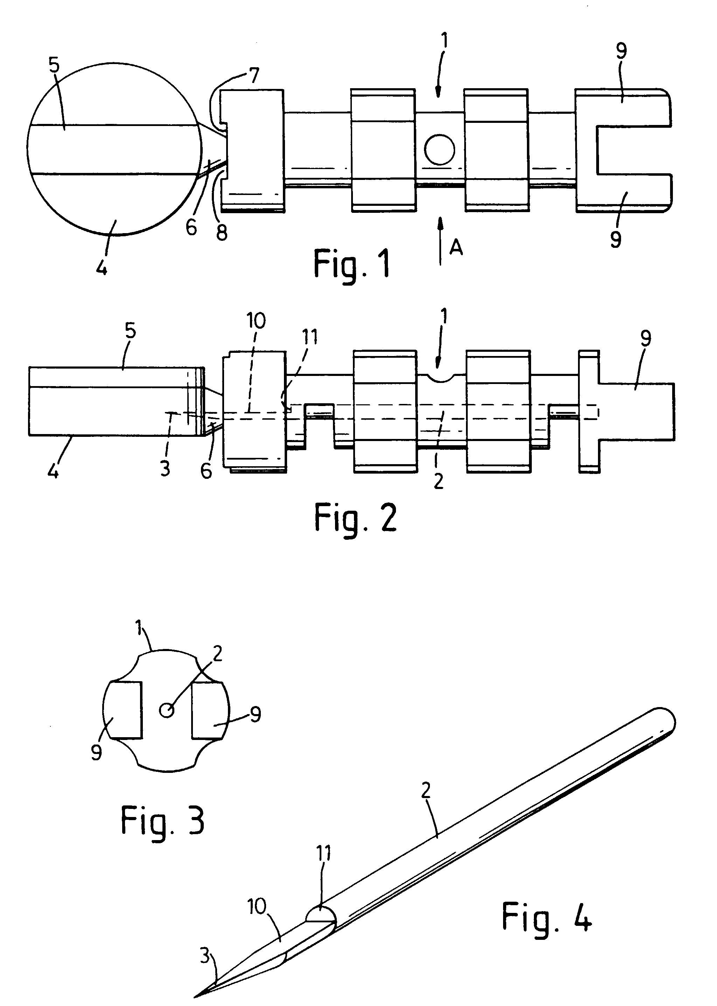

The lancet has an elongate body 1 of moulded plastics material. It resembles the spool of a spool valve, having alternating large and small diameter sections. Co-axially encased within it there is a needle 2, indicated in broken lines in Figure 2, which projects in a sharp tip 3 at the forward end.

This tip is initially encased in a cap 4 which is in the form of a disc with a diametral rib 5. This could be shortened to a central stud. The cap is integrally moulded with the body 1 and connects to it by a neck 6 which will be weak enough to be sheared off from end surface 7 by a twisting action. The forward end of the body 1 has a transverse recess 8 in which the surface 7 forms the base.

At the other end, the body 1 is extended by two opposed wings 9 projecting rearwardly. In the initial state, the rear end portion of the needle 2 is fully embedded in the body with its end face aligned with the roots of these wings.

The larger diameter portions of the body 1, at least towards the rear, are not smoothly cylindrical on their outer surfaces. They have indentations as best seen in Figure 3. This is to afford a grip for formations within the firing device to stop the body 1 rotating about its axis when the cap 4 is twisted off.

Referring to Figure 4, the needle 2 is cylindrical over most of its length. But at its forward end a flat 10 is formed, conveniently by grinding, before or during the operation to produce the sharp tip 3. This flat extends back from the extremity of the tip to a radial, semicircular shoulder 11.

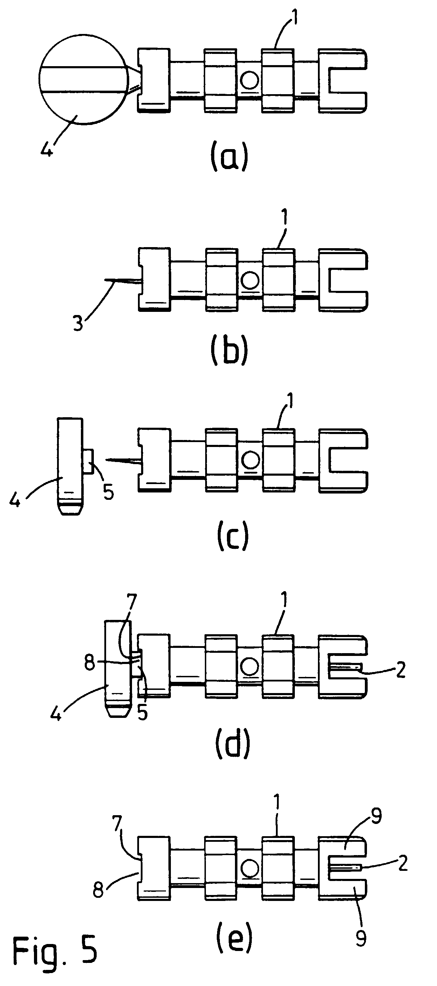

Referring to Figure 5, stage (a) shows the lancet in its pre-use state, as in Figures 1 and 2. The cap 4 is then twisted off to leave the needle tip 3 exposed as in stage (b). The pricker is then used. Although the needle 2 is slidable within the body 1 there is considerable friction preventing this. The resistance of the user's skin to the sharp tip will be substantially less than the force needed to shift the needle relative to the body, and so for the actual pricking operation the needle will remain fast with the body.

After use, the cap 4 is offered up as in stage (c) with its rib 5 towards the forward end of the lancet body 1. It is then pressed against it to achieve stage (d). Here, the rib 5 has pushed the needle rearwardly and entered the recess 8, so the tip of the needle is flush with the surface 7 and thus safe. At the same time, the rear end of the needle 2 projects, but is shielded between the wings 9. The cap can then be thrown away, and so can the lancet be discarded, it now being in the state illustrated at stage (e).

It will be appreciated that the shoulder 11 will stop the needle 2 being drawn forward out of the body 1 when the cap is removed, but will not impede the needle being pushed backwards.

Instead of relying on friction to hold the needle in the forward position during the pricking operation, other measures could be taken. For example, the body moulding could form a thin plastics membrane over the rear end of the needle. This would be strong enough to remain unbroken while the skin was being pricked, but weak enough to be pierced by the rear end of the needle when the cap was pushed against the tip. Another way would be to make a very small flat at the rear end of the needle, whose main length could be forced through the resultant slightly narrowed throat formed by the flat.

## Claims

1. A lancet for a skin pricker comprising a needle (2) with a tip (3) and an elongate body (1) of moulded plastics material encasing most of the needle (2), which is movable rearwardly with respect to the body (1) to retract the needle tip (3) within the body (1) after use, characterised in that the needle tip (3) is initially encased by a breakaway cap (4) integrally moulded with the body at its forward end and which is exposed when the cap (4) is removed, in that the mutual engagement of the needle (2) and body (1) is such that the needle (2) cannot move forwards from its initial position relative to the body (1) and in that the needle (2) and body (1) remain effectively integral and move together during the act of skin pricking.

2. A lancet as claimed in Claim 1, characterised in that the mutual engagement is a frictional grip.

3. A lancet as claimed in Claim 1, characterised in that the mutual engagement is a membrane at the rear end of the body (1) against which the rear end of the needle (2) bears during skin pricking, but which can be broken when the needle (2) is urged rearwardly by a substantial force.

4. A lancet as claimed in Claim 1, characterised in that the mutual engagement is a throat at the rear end of the body (1) in which the rear end of the needle (2) is trapped during skin pricking, but through which that rear end of the needle (2) can be urged by a substantial force.

5. A lancet as claimed in any preceding Claim, wherein the cap (4) is adapted to serve as an implement for pushing the tip (3) of the needle, after use, back into the body (1).

6. A lancet as claimed in Claim 5, characterised in that the forward end of the body (1) is recessed and the cap (4) has a projection (6) which can enter the recess (8) when pushing the tip of the needle.

7. A lancet as claimed in any preceding Claim, characterised in that the rear end of the body (1) is adapted to shield the rear end of the needle (2) when the needle is retracted.

8. A lancet as claimed in any preceding claim, characterised in that the impediment to forward movement of the needle (2) from its initial position relative to the body (1) is a forwardly facing shoulder (11) where the needle (2) reduces in cross-section to a leading length portion (10) projecting from the body (1) and terminating in the tip (3).

9. A lancet as claimed in Claim 8, wherein the needle (2) is generally cylindrical with a flat (10) formed over the leading length portion to make the reduced cross section.

## Patentansprüche

1. Lanzette für einen Hautstecher, der eine Nadel (2) mit einer Spitze (3) und einen langgestreckten Körper (1) aus ausgeformten Kunststoff aufweist, der den größten Teil der Nadel (2) umschließt, welche bezüglich des Körpers (1) nach hinten bewegbar ist, um die Nadelspitze (3) in den Körper (1) nach Gebrauch zurückzuziehen, dadurch gekennzeichnet, daß die Nadelspitze (3) anfänglich von einer in einem Stück mit dem Körper an seinem vorderen Ende angeformten wegbrechbaren Kappe (4) umschlossen ist und freigesetzt wird, wenn die Kappe (4) entfernt wird, daß der gegenseitige Eingriff von Nadel (2) und Körper (1) derart beschaffen ist, daß sich die Nadel (2) nicht nach vorne aus ihrer Anfangstellung bezüglich des Körpers (1) bewegen kann, und daß die Nadel (2) und der Körper (1) effektiv ein Stück bleiben und sich zusammen während des Hautdurchstechungsvorgangs bewegen.

2. Lanzette nach Anspruch 1, dadurch gekennzeichnet, daß der gegenseitige Eingriff durch einen Reibgriff erreicht ist.

3. Lanzette nach Anspruch 1, dadurch gekennzeichnet, daß der gegenseitige Eingriff durch eine am hinteren Ende des Körpers (1) vorgesehene Membran erreicht wird, gegen die das hintere Ende der Nadel (2) während des Durchstechens der Haut drückt, die jedoch durchbrochen werden kann, wenn die Nadel (2) nach rückwärts durch eine starke Kraft gedrückt wird.

4. Lanzette nach Anspruch 1, dadurch gekennzeichnet, daß der gegenseitige Eingriff durch eine am hinteren Ende des Körpers (1) vorgesehene Verengung erreicht wird, in welcher das hintere Ende der Nadel (2) während des Durchstechens der Haut eingeschlossen ist, durch die jedoch das hintere Ende der Nadel (2) durch eine starke Kraft hindurchgedrückt werden kann.

5. Lanzette nach einem vorhergehenden Anspruch, bei welchem die Kappe (4) als Werkzeug zum Drücken der Spitze (3) der Nadel nach Gebrauch zurück in den Körper (1) einsetzbar ist.

6. Lanzette nach Anspruch 5, dadurch gekennzeichnet, daß das vordere Ende des Körpers (1) ausgespart ist und daß die Kappe (4) einen Vorsprung (6) hat, der in die Aussparung (8) eintreten kann, wenn gegen die Spitze der Nadel gedrückt wird.

7. Lanzette nach einem vorhergehenden Anspruch, dadurch gekennzeichnet, daß das hintere Ende des Körpers (1) das hintere Ende der Nadel (2) schützen kann, wenn die Nadel zurückgezogen ist.

8. Lanzette nach einem vorhergehenden Anspruch, dadurch gekennzeichnet, daß das Hindernis für eine Vorwärtsbewegung der Nadel (2) aus ihrer Anfangsposition bezüglich des Körpers (1) eine nach vorne weisende Schulter (11) ist, wo sich die Nadel (2) im Querschnitt zu einem vorderen Längenabschnitt (10) verringert, der aus dem Körper (1) vorsteht und in der Spitze (3) endet.

9. Lanzette nach Anspruch 8, bei welcher die Nadel (2) insgesamt zylindrisch ist und eine Abflachung (10) über dem vorderen Längenabschnitt ausgebildet ist, um den reduzierten Querschnitt zu erzielen.

## Revendications

1. Lancette pour un autopiqueur cutané, comprenant une aiguille (2) ayant une pointe (3) et un long corps (1) constitué de matière plastique moulée, dans lequel est encastrée la majeure partie de l'aiguille (2), cette dernière pouvant être déplacée vers l'arrière par rapport au corps (1) de façon à rétracter la pointe (3) de l'aiguille à l'intérieur du corps (1) après l'utilisation, caractérisée par le fait que la pointe (3) de l'aiguille est initialement enfermée par un capuchon cassable (4), moulé d'un seul tenant avec le corps au niveau de son extrémité avant, et est exposée lorsque le capuchon (4) est enlevé, par le fait que la prise mutuelle entre l'aiguille (2) et le corps (1) est telle que l'aiguille (2) ne peut pas se déplacer vers l'avant depuis sa position initiale par rapport au corps (1), et par le fait que l'aiguille (2) et le corps (1) restent efficacement solidaires et se déplacent ensemble pendant l'action de piquage de la peau.

2. Lancette selon la revendication 1, caractérisée par le fait que la prise mutuelle est un accrochage par friction.

3. Lancette selon la revendication 1, caractérisée par le fait que la prise mutuelle est une membrane aménagée au niveau de l'extrémité arrière du corps (1), contre laquelle l'extrémité arrière de l'aiguille (2) s'appuie pendant le piquage de la peau, mais qui peut être cassée lorsque l'aiguille (2) est poussée vers l'arrière avec une force substantielle.

4. Lancette selon la revendication 1, caractérisée par le fait que la prise mutuelle est une gorge aménagée au niveau de l'extrémité arrière du corps (1), dans laquelle l'extrémité arrière de l'aiguille (2) est emprisonnée pendant le piquage de la peau, mais au travers de laquelle l'extrémité arrière de l'aiguille (2) peut être poussée par une force substantielle.

5. Lancette selon l'une quelconque des revendications précédentes, dans laquelle le capuchon (4) est agencé pour servir d'outil pour repousser la pointe (3) de l'aiguille dans le corps (1) après l'utilisation.

6. Lancette selon la revendication 5, caractérisée par le fait que l'extrémité avant du corps (1) est évidée et le capuchon (4) possède une partie saillante (6) qui peut pénétrer dans l'évidement (8) lorsque la pointe de l'aiguille est poussée.

7. Lancette selon l'une quelconque des revendications précédentes, caractérisée par le fait que l'extrémité arrière du corps (1) est adaptée pour protéger l'extrémité arrière de l'aiguille (2) lorsque l'aiguille est rétractée.

8. Lancette selon l'une quelconque des revendications précédentes, caractérisée par le fait que l'obstacle au déplacement de l'aiguille (2) vers l'avant vis-à-vis de sa position initiale par rapport au corps (1) est un épaulement (11) dirigé vers l'avant, à l'endroit où la section transversale de l'aiguille (2) diminue sur une partie de longueur avant (10) faisant saillie du corps (1) et se terminant en pointe (3).

9. Lancette selon la revendication 8, dans laquelle l'aiguille (2) est généralement cylindrique, avec un plat (10) formé sur la partie de longueur avant, pour réaliser la section transversale réduite.
